# EUROPÄISCHE PATENTSCHRIFT

(11) **EP 2 716 208 B1**
(45) Veröffentlichungstag und Bekanntmachung des Hinweises auf die Patenterteilung: **12.09.2018**
(21) Anmeldenummer: 13401110.5
(22) Anmeldetag: 01.10.2013
(51) Int. Cl.: A61B 1/12, B08B 9/032

(54) **Reinigungsvorrichtung für englumige Kanäle, Rohre, Leitungen oder dergleichen**
Cleaning device for narrow-necked channels, pipes, lines or the like
Dispositif de nettoyage de canaux, tuyaux, conduites étroits ou analogues

(30) Priorität: 05.10.2012 DE 102012109463
(43) Veröffentlichungstag der Anmeldung: 09.04.2014
(73) Patentinhaber: Miele & Cie. KG, 33332 Gütersloh (DE)
(72) Erfinder: Buhl, David, 33613 Bielefeld (DE); Haße, Julian, 33104 Paderborn (DE); Malec, Tobias, 33729 Bielefeld (DE); Marks, Volker, 33611 Bielefeld (DE); Rummler, Britta, 33604 Bielefeld (DE)

(56) Entgegenhaltungen:
- EP-A1- 2 065 059
- DE-C2- 3 308 729
- DE-U1- 29 620 011
- US-A- 5 408 991
- US-A1- 2005 079 094

## Beschreibung

Die Erfindung betrifft eine Reinigungsvorrichtung für englumige Kanäle, Rohre, Leitungen oder dergleichen, mit einer einen Mediumseingang und einen Mediumsausgang aufweisenden Verteilkammer, einer Reinigungsflüssigkeitszuführungsleitung und einer Druckluftzuführungsleitung, wobei die Verteilkammer eingangsseitig an die Reinigungsflüssigkeitszuführungsleitung angeschlossen ist.

Englumige Kanäle, Rohre, Leitungen oder dergleichen im Sinne der Erfindung sind Kanäle, Rohre, Leitungen oder dergleichen mit einem vergleichsweise kleinen inneren Querschnitt, das heißt einer vergleichsweise kleinen lichten Weite. Hierunter fallen insbesondere medizinische Instrumente, wie zum Beispiel Kanülen, Katheter, Endoskope und/oder dergleichen.

Zur Reinigung englumiger Kanäle, Rohre, Leitungen oder dergleichen ist es aus dem Stand der Technik bekannt geworden, diese mit einer Reinigungsflüssigkeit zu beschicken. Infolge des Hindurchströmens der Reinigungsflüssigkeit durch die englumigen Kanäle, Rohre, Leitungen oder dergleichen hindurch kommt es zu einem Abtrag der an der Innenoberfläche der englumigen Kanäle, Rohre, Leitungen oder dergleichen unter Umständen anhaftenden Schmutz- und/oder Fremdpartikel.

Das einfache Hindurchleiten von Reinigungsflüssigkeit durch zu reinigende englumige Kanäle, Rohre, Leitungen oder dergleichen kann nur bedingt für eine zufriedenstellende Reinigung sorgen. Es ist deshalb aus dem Stand der Technik bekannt geworden, die zur Reinigung vorgesehene Reinigungsflüssigkeit unter erhöhtem Druck durch die zu reinigenden englumigen Kanäle, Rohre, Leitungen und/oder dergleichen hindurchzuführen. Das Reinigungsergebnis kann so verbessert werden, ist im Ergebnis aber immer noch nicht zufriedenstellend, insbesondere dann nicht, wenn an der Innenoberfläche von zu reinigenden englumigen Kanälen, Rohren, Leitungen oder dergleichen anhaftende Schmutz- oder Fremdpartikel eingetrocknet sind.

Zur weiteren Verbesserung des Reinigungsergebnisses ist mit der DE 33 08 729 C2 eine Reinigungsvorrichtung der gattungsgemäßen Art vorgeschlagen worden. Diese vorbekannte Reinigungsvorrichtung verfügt über eine Verteilkammer, die der Lehre der DE 33 08 729 C2 nach als Mischkammer dient. Diese Mischkammer ist zur Zuführung von Reinigungsflüssigkeit einerseits an eine Reinigungsflüssigkeitszuführungsleitung und zur Zuführung von Druckluft andererseits an eine Druckluftzuführungsleitung angeschlossen. Im Betriebsfall findet innerhalb der als Mischkammer dienenden Verteilkammer eine Vermischung von Reinigungsflüssigkeit und Druckluft statt, und es wird im Weiteren dieses Reinigungsflüssigkeit-Luft-Gemisch zur Reinigung der englumigen Kanäle, Rohre, Leitungen oder dergleichen in diese eingeleitet. Wenn das Reinigungsflüssigkeit-Luft-Gemisch durch die zu reinigenden Kanäle, Leitungen oder dergleichen geführt wird, erleiden die inneren Oberflächen dieser Kanäle, Rohre, Leitungen oder dergleichen eine Erschütterung an den Phasengrenzen, an welchen die Luftphase sich zu der Flüssigkeitsphase ändert und umgekehrt. Aufgrund dieser durch das eingeleitete Reinigungsflüssigkeit-Luft-Gemisch bewirkter Erschütterungen stellt sich ein nochmals verbessertes Reinigungsergebnis ein Aus der US 2005/0079094 A1 ist darüber hinaus eine Mess- und Prüfeinrichtung zur Kontrolle des Durchflusses einer Reinigungsflüssigkeit durch die Kanäle von Endoskopen bekannt. Diese weist eine außerhalb eines Spülraums angeordnete Verteilkammer auf, mit der ein fest vorgegebenes Volumen an Reinigungsflüssigkeit bereitgestellt wird, die mittels Druckluft durch einen Kanal eines Endoskops gedrückt wird. Dadurch wird für jeden Kanal einzeln die Messung des Volumenstroms mittels einer Messung der Zeitdauer für die Entleerung der Verteilkammer ermöglicht. Obgleich sich die aus dem Stand der Technik vorbekannten Vorrichtungen zur Reinigung englumiger Kanäle, Rohre, Leitungen oder dergleichen im alltäglichen Praxiseinsatz bewährt haben, besteht weiterhin Verbesserungsbedarf. So sind die für die wünschenswerter Weise zu erzielende Reinigungswirkung relevanten Parameter in hohem Maße von der geometrischen Ausgestaltung der zu reinigenden Kanäle, Rohre, Leitungen oder dergleichen abhängig. Insofern können vorrichtungsseitig fest vorgegebene Reinigungsparameter in ungünstigen Fällen dazu führen, dass einzelne Kanäle, Rohre, Leitungen oder dergleichen gar nicht, zumindest aber nicht hinreichend gut gereinigt werden. Darüber hinaus stellen hartnäckige Verschmutzungen und Verkrustungen nach wie vor ein Problem dar und lassen sich auch mit den aus dem Stand der Technik vorbekannten Reinigungsvorrichtungen nicht immer in zufriedenstellendem Maße entfernen.

Es ist deshalb die **Aufgabe** der Erfindung, eine gattungsgemäße Reinigungsvorrichtung vorzuschlagen, die es gestattet, ein in wünschenswerter Weise zu erzielendes Reinigungsergebnis zu erreichen. Darüber hinaus soll mit der Erfindung ein Verfahren zur Reinigung von englumigen Kanälen, Rohren, Leitungen oder dergleichen vorgeschlagen werden.

Vorrichtungsseitig wird zur Lösung der Erfindung eine Reinigungsvorrichtung der eingangs genannten Art vorgeschlagen, die sich dadurch auszeichnet, dass die Verteilkammer ausgangsseitig Verbindungsanschlüsse zur auswechselbaren Anordnung von zu reinigenden englumigen Kanälen, Rohren, Leitungen oder dergleichen aufweist, wobei die Verbindungsanschlüsse jeweils einen gegenüber dem Durchströmungsquerschnitt der Reinigungsflüssigkeitszuführungsleitung verringerten Durchströmungsquerschnitt aufweisen, und dass die Druckluftzuführungsleitung in die Reinigungsflüssigkeitszuführungsleitung einmündet.

Die nach der Erfindung vorgesehene Verteilkammer verfügt ausgangsseitig über eine Mehrzahl von Verbindungsanschlüssen. Diese dienen der auswechselbaren Anordnung von zu reinigenden englumigen Kanälen, Rohren, Leitungen oder dergleichen. Über die eingangsseitig an die Verteilkammer angeschlossene Reinigungsflüssigkeitszuführungsleitung strömt im bestimmungsgemäßen Verwendungsfall Reinigungsflüssigkeit in die Verteilkammer ein, von wo aus dann eine Verteilung der Reinigungsflüssigkeit auf die einzelnen über die Verbindungsanschlüsse an die Verteilkammer angeschlossenen Kanäle, Rohre, Leitungen oder dergleichen stattfindet. Dabei wirken die an die Verteilkammer angeschlossenen Kanäle, Rohre, Leitungen oder dergleichen als ein gemeinsamer hydraulischer Widerstand, was eine vereinfachte Einstellung der Reinigungsparameter gestattet. Es findet insbesondere eine einheitliche Druckverteilung statt, so dass eine Beeinflussung des Reinigungsergebnisses infolge unterschiedlicher geometrischer Ausgestaltungen der an die Verteilkammer angeschlossenen Kanäle, Rohre, Leitungen oder dergleichen unterbunden ist.

Es ist erfindungsgemäß vorgesehen, dass die Verbindungsanschlüsse jeweils einen gegenüber dem Durchströmungsquerschnitt der Reinigungsflüssigkeitsfzuführungsleitung verringerten Durchströmungsquerschnitt aufweisen. Es wird hierdurch sichergestellt, dass es nicht ungewollt zu einem Abbau des in der Reinigungsflüssigkeit herrschenden Flüssigkeitsdrucks innerhalb der Verteilkammer kommt. Es kann sich vielmehr ein Staudruck innerhalb der Verteilkammer aufbauen, so dass die Reinigungsflüssigkeit ausgehend von der Verteilkammer mit einem erhöhten Druck durch die zu reinigenden Kanäle, Rohre, Leitungen oder dergleichen hindurchgeführt wird.

Die Druckluftzuführungsleitung mündet erfindungsgemäß in die Reinigungsflüssigkeitszuführungsleitung ein. Sobald im bestimmungsgemäßen Verwendungsfall über die Druckluftzuführungsleitung ein Drucklufteintrag in die Reinigungsflüssigkeitszuführungsleitung stattfindet, bedingt dies einen abrupten, stoßartigen Anstieg des Reinigungsflüssigkeitsdrucks. Aufgrund der im Durchströmungsquerschnitt im Vergleich zum Durchströmungsquerschnitt der Reinigungsflüssigkeitszuführungsleitung verringert ausgebildeten Verbindungsanschlüsse kann es sogar kurzzeitig zu einem Druckanstieg innerhalb der Reinigungsflüssigkeit kommen, der oberhalb des Drucks der zugeführten Druckluft liegt. Es wird so ein massiver Druckstoß erzeugt, der auch hartnäckige Verkrustungen und Verschmutzungen innerhalb der englumigen Kanäle, Rohre, Leitungen oder dergleichen abzulösen vermag, so dass insgesamt ein verbessertes Reinigungsergebnis erzielt wird. Dies gilt insbesondere auch für solche Kanäle, Rohre, Leitungen oder dergleichen, die Ringspalte bereitstellen und im Besonderen zur Verstopfung neigen und deren Reinigung mit vorbekannten Reinigungsvorrichtungen stets problematisch ist.

Die mit der erfindungsgemäßen Reinigungsvorrichtung vorgesehene Drucklufteinspeisung dient im Unterschied zur vorbekannten Reinigungsvorrichtung nach der DE 33 08 729 C2 nicht dazu, innerhalb einer Mischkammer ein Reinigungsflüssigkeit-Luft-Gemisch herzustellen. Es ist erfindungsgemäß vielmehr vorgesehen, dass die Druckluftzuführungsleitung in die Reinigungsflüssigkeitszuführungsleitung einmündet, was es bei einem Drucklufteintrag gestattet, einen Druckpuls innerhalb der Reinigungsflüssigkeit zu erzeugen, so dass diese mit entsprechend erhöhtem Druck durch die zu reinigenden Kanäle, Rohre, Leitungen oder dergleichen pulsweise geführt werden kann. Dies bedingt das mit der erfindungsgemäßen Vorrichtung bewirkte gute Reinigungsergebnis.

Gemäß einer Alternative der Erfindung wird die Druckluft nicht schlagartig in die die Reinigungsflüssigkeit führende Reinigungsflüssigkeitszuführungsleitung eingelassen. Es erfolgt vielmehr bis zum Erreichen eines Maximaldrucks eine Drucklufteinleitung mit wachsendem Druck. Ein solcher sanfter Druckanstieg anstelle eines stoßartigen Druckanstiegs führt im Ergebnis zu einer deutlich geringeren mechanischen Belastung der zu reinigenden Kanäle, Rohre, Leitungen oder dergleichen und bietet sich deshalb insbesondere dann an, wenn die zu reinigenden Kanäle, Rohre, Leitungen oder dergleichen mechanisch zu schonen sind, beispielsweise zur Vermeidung eines unnötigen Verschleiß.

Es ist gemäß einem weiteren Merkmal der Erfindung vorgesehen, dass die Druckluftzuführungsleitung ein Rückschlagventil aufweist, das in Durchströmungsrichtung der Druckluftzuführungsleitung vor der Einbindung der Druckluftzuführungsleitung in die Reinigungsflüssigkeitszuführungsleitung positioniert ist. Dieses Rückschlagventil dient zur Vermeidung einer Rückströmung von Reinigungsflüssigkeit in die Druckluftzuführungsleitung. Es dient mithin dem sicheren Betrieb der erfindungsgemäßen Reinigungsvorrichtung.

Gemäß einem weiteren Merkmal der Erfindung ist vorgesehen, dass die Reinigungsflüssigkeitszuführungsleitung über ein Rückschlagventil verfügt, das in Durchströmungsrichtung der Reinigungsflüssigkeitszuführungsleitung vor der Einmündung der Druckluftzuführungsleitung in die Reinigungsflüssigkeitszuführungsleitung positioniert ist. Auch dieses Rückschlagventil dient einem sicheren Betrieb der erfindungsgemäßen Reinigungsvorrichtung, da es vermeidet, dass bei einströmender Druckluft in die Reingungsflüssigkeitszuführungsleitung Druckluft in eine der zur Eingangsseite der Verteilkammer weisenden Richtung entgegengesetzten Richtung strömt. Insbesondere eine die Reinigungsflüssigkeit fördernde Pumpe kann so vor einer Beschädigung durch eindringende Druckluft geschützt werden.

Gemäß einem weiteren Merkmal der Erfindung ist vorgesehen, dass die Druckluftzuführungsleitung ein Einlassventil aufweist, das in Durchströmungsrichtung der Druckluftzuführungsleitung dem Rückschlagventil vorgeschaltet ist. Dieses Einlassventil dient der gesteuerten Zuführung von Druckluft in die Reinigungsflüssigkeitszuführungsleitung. Bei geschlossenem Einlassventil ist die Druckluftzuführungsleitung versperrt. Die Verteilkammer erreicht in diesem Fall allein Reinigungsflüssigkeit. Sobald das Einlassventil geöffnet ist, strömt unter Passierung des Rückschlagventils Druckluft in die Reinigungsflüssigkeitszuführungsleitung, was in der Konsequenz zu dem schon vorbeschriebenen Druckstoß innerhalb der Reinigungsflüssigkeit führt. Im bestimmungsgemäßen Verwendungsfall ist das Einlassventil für die Dauer einer vorgebbaren Zeitspanne geöffnet, wodurch sich der Druckimpuls in seiner zeitlichen Ausdehnung bestimmen lässt. Durch wiederholtes Öffnen und Schließen des Einlassventils bestimmt sich die Frequenz der sich infolge des Drucklufteintrags innerhalb der Reinigungsflüssigkeit auf- und abbauenden Druckstöße.

Gemäß einem weiteren Merkmal der Erfindung ist vorgesehen, dass die Reinigungsflüssigkeitszuführungsleitung einen Drucksensor aufweist, der in Durchströmungsrichtung der Reinigungsflüssigkeitszuführungsleitung der Einmündung der Druckluftzuführungsleitung in die Reinigungsflüssigkeitszuführungsleitung nachgeschaltet ist.

Dieser Drucksensor dient der Erfassung des innerhalb der Reinigungsflüssigkeitszuführungsleitung herrschenden Drucks, womit sich die im Falle eines Drucklufteintrags ergebende Druckdifferenz zwischen dem vor der Einmündungsstelle und dem nach der Einmündungsstelle der Druckluftzuführungsleitung in der Reinigungsflüssigkeitszuführungsleitung herrschenden Druck ermitteln lässt.

Auf Basis der ermittelten Druckdifferenz kann ein optimierter Betrieb der erfindungsgemäßen Reinigungsvorrichtung durchgeführt werden. So kann ein neuer Druckimpuls nur dann eingeleitet werden, wenn der durch einen vorangegangenen Druckimpuls angestiegene Flüssigkeitsdruck wieder abgebaut hat. Andernfalls könnte es zu Rückströmeffekten innerhalb der Reinigungsflüssigkeitszuführungsleitung und damit unter Umständen zu Beschädigungen einzelner Bauteile der Reinigunsvorrichtung kommen. Die Druckdifferenzbestimmung dient insofern der Bereitstellung einer optimierten Druckstoßfrequenz. Alternativ zu einer Druckdifferenzsteuerung kann auch eine Zeitsteuerung vorgesehen sein, die sich aus der Dauer des Druckimpulses einerseits und der Dauer des sich nach einem Druckimpuls ergebenden Druckabfalls errechnen lässt.

Verfahrensseitig wird zur **Lösung** der vorstehenden Aufgabe vorgeschlagen ein Verfahren zur Reinigung von englumigen Kanälen, Rohren, Leitungen oder dergleichen, bei dem die zu reinigenden Kanäle, Rohre, Leitungen oder dergleichen mittels einer gemeinsamen Verteilkammer gleichzeitigt mit einer einen vorgebbaren Flüssigkeitsdruck aufweisenden Reinigungsflüssigkeit beschickt werden, bei dem in die die Reinigungsflüssigkeit führende Reinigungsflüssigkeitszuführungsleitung über die Dauer einer vorgebbaren Zeitspanne Druckluft mit einem oberhalb des Flüssigkeitsdruck liegenden Luftdruck eingetragen wird und bei dem ein wiederholter Drucklufteintrag in die die Reinigungsflüssigkeit führende Reinigungsflüssigkeitszuführungsleitung erfolgt, sobald der infolge des vorangegangenen Drucklufteintrags erhöhte Flüssigkeitsdruck wieder abgebaut ist.

Das erfindungsgemäße Verfahren zeichnet sich dadurch aus, dass für eine zeitweise Erhöhung des Flüssigkeitsdrucks in die die Reinigungsflüssigkeit führende Reinigungsflüssigkeitszuführungsleitung Druckluft eingetragen wird. Dabei erfolgt der Drucklufteintrag stoßweise, was zu Druckstößen auch innerhalb der Reinigungsflüssigkeit führt. Dabei ist mit dem erfindungsgemäßen Verfahren vorgesehen, dass ein wiederholter Drucklufteintrag stattfindet, das heißt eine gepulste Erhöhung des Flüssigkeitsdrucks durchgeführt wird. Dabei ist von verfahrenswesentlicher Bedeutung, dass ein wiederholter Drucklufteintrag erst dann stattfindet, wenn der durch den vorangegangenen Drucklufteintrag bedingte Anstieg des Flüssigkeitsdrucks wieder abgebaut ist.

Zu diesem Zweck kann gemäß einem weiteren Merkmal der Erfindung eine Druckdifferenzbestimmung oder eine Zeitberechnung durchgeführt werden. In vorteilhafter Weise kann so ein optimierter Verfahrensbetrieb erreicht werden, wie vorstehend schon anhand der erfindungsgemäßen Vorrichtung erläutert.

Weitere Vorteile und Merkmale der Erfindung ergeben sich aus der nachfolgenden Beschreibung anhand der Figuren. Dabei zeigen
- Fig. 1: In schematischer Darstellung die erfindungsgemäße Reinigungsvorrichtung;
- Fig. 2: in einer schematischen Diagrammdarstellung die erfindungsgemäße Verfahrensdurchführung gemäß einer ersten Alternative
- Fig. 3: in einer schematischen Diagrammdarstellung die erfindungsgemäße Verfahrensdurchführung gemäß einer zweiten Alternative.

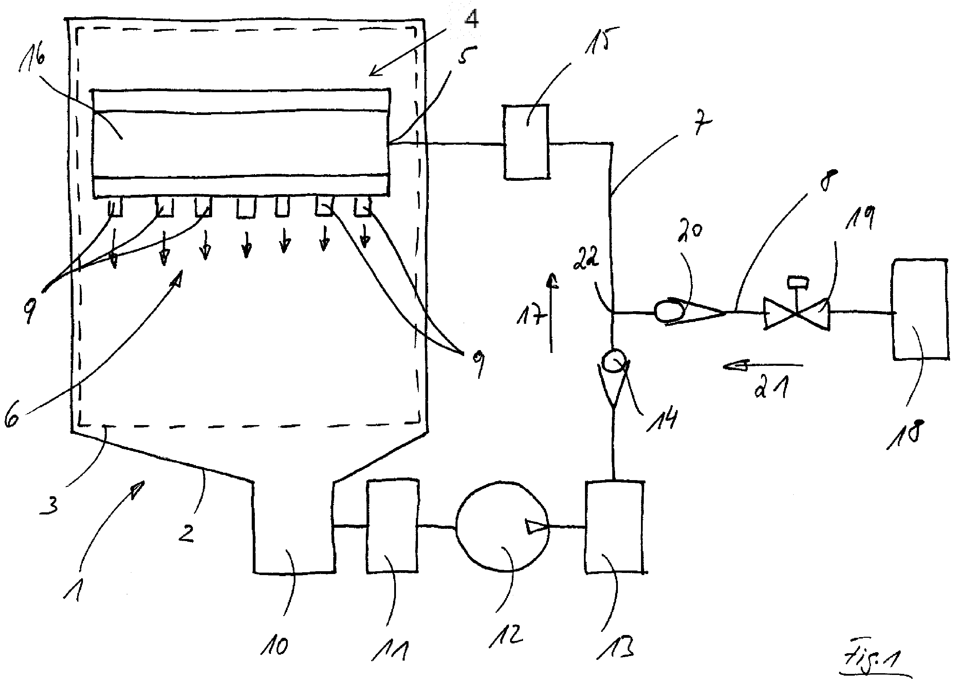

Fig. 1 lässt die erfindungsgemäße Reinigungsvorrichtung 1 in einer schematischen Darstellung erkennen.

Die Reinigungseinrichtung 1 verfügt über ein Gehäuse 2. Innerhalb des Gehäuses 2 ist ein Spülraum 3 ausgebildet, der eine Verteilkammer 4 aufnimmt. Die Verteilkammer 4 weist einen Mediumseingang 5 auf, der an einer Reinigungsflüssigkeitszuführungsleitung 7 angeschlossen ist.

Die Verteilkammer 4 weist einen Mediumsausgang 6 auf, der über eine Vielzahl einzelner Verbindungsanschlüsse 9 verfügt. Im bestimmungsgemäßen Verwendungsfall dienen diese Verbindungsanschlüsse 9 der auswechselbaren Anordnung von zu reinigenden englumigen Kanälen, Rohren, Leitungen oder dergleichen, beispielsweise von MIC-Instrumenten.

Das Gehäuse 2 der Reinigungseinrichtung 1 stellt einen Sammeltopf 10 bereit. Im bestimmungsgemäßen Verwendungsfall sammelt sich hier aus der Verteilkammer 4 über die Verbindungsanschlüsse 9 und die zu reinigenden Kanäle, Rohre, Leitungen oder dergleichen geführte Reinigungsflüssigkeit an. Der Sammeltopf ist seinerseits an die Reinigungsflüssigkeitszuführungsleitung 7 angeschlossen, die eingangsseitig der Verteilkammer 4 an deren Mediumseingang 5 angeschlossen ist.

Im bestimmungsgemäßen Verwendungsfall wird die sich im Sammeltopf 10 der Reinigungsvorrichtung 1 ansammelnde Reinigungsflüssigkeit über eine Pumpe 12 durch die Reinigungsflüssigkeitszuführungsleitung 7 gefördert. Im gezeigten Ausführungsbeispiel sind mehrere Filter 11, 13, 15 und 16 vorgesehen, die die Reinigungsflüssigkeit auf ihrem Weg durch die Reinigungsflüssigkeitszuführungsleitung 7 passiert. Es genügt aber, wenn ein oder eine Teilmenge der gezeigten Filter 11, 13, 15 und 16 vorhanden ist. Die Reinigungsflüssigkeit wird dabei in Richtung des Pfeils 17 durch die Reinigungsflüssigkeitszuführungsleitung 7 hindurchgeführt.

Die erfindungsgemäße Reinigungsvorrichtung 1 verfügt desweiteren über eine Druckluftzuführungsleitung 8. Diese mündet bei der Einmündung 22 in die Reinigungsflüssigkeitszuführungsleitung 7 ein. Dabei sind in Durchströmungsrichtung der Eimündung 22 sowohl seitens der Reinigungsflüssigkeitszuführungsleitung 7 als auch seitens der Druckluftzuführungsleitung 8 jeweils ein Rückschlagventil 14 beziehungsweise 20 vorgeschaltet.

Die Druckluftzuführungsleitung 8 ist anderendseitig an eine Druckluftquelle 18 angeschlossen. In Richtung des Pfeils 21 kann der Druckluftquelle 18 entstammende Druckluft über das Einlassventil 19 und das Rückschlagventil 20 an der Einmündung 22 in die Reinigungsflüssigkeitszuführungsleitung 7 eingetragen werden.

Im bestimmungsgemäßen Verwendungsfall wird über die Pumpe 12 Reinigungsflüssigkeit gefördert, die in Richtung des Pfeils 17 die Reinigungsflüssigkeitszuführungsleitung 7 durchströmt. Sie mündet über den Mediumseingang 5 in die Verteilkammer 4 ein, von wo aus sie über die einzelnen Verbindungsanschlüsse 9 verteilt und in die daran angeschlossenen Kanäle, Rohre, Leitungen oder dergleichen zwecks Reinigung derselben einströmt. Der Flüssigkeitsdruck beträgt typischerweise 0,5 bar.

Zu einem vorgebbaren Zeitpunkt wird das Einlassventil 19 geöffnet. In der Konsequenz strömt Druckluft in Entsprechung des Pfeils 21 durch die Druckluftzuführungsleitung 8 und wird bei der Einmündung 22 in die Reinigungsflüssigkeitszuführungsleitung 7 eingetragen. Infolge dessen kommt es zu einem Druckstoß innerhalb der Reinigungsflüssigkeit, der sich über die Reinigungsflüssigkeitszuführungsleitung 7, die Verteilkammer 4 und die Verbindungsanschlüsse 9 bis in die zu reinigenden Kanäle, Rohre, Leitungen oder dergleichen fortpflanzt. Infolge dessen kann ein Abtrag auch fest an der Innenoberfläche der zu reinigenden Kanäle, Rohre, Leitungen oder dergleichen anhaftender Verschmutzungen erreicht werden.

Die zur Verteilkammer 4 hinführende Reinigungsflüssigkeitszuführungsleitung 7 hat einen deutlich größeren Innendurchmesser als die von der Verteilkammer 4 abführenden Verbindungsanschlüsse 9. So weist die Reinigungsflüssigkeitszuführungsleitung 7 beispielsweise einen Innendurchmesser auf, der zwischen 5 mm und 30 mm liegt. Die Verbindungsanschlüsse weisen indes jeweils einen Innendurchmesser auf, der zwischen 0,1 mm und 6 mm beträgt. Dabei beträgt der Innendurchmesser der Reinigungsflüssigkeitszuführungsleitung 7 vorzugsweise ein Mehrfaches, insbesondere das 3 bis 20-fache, des Innendurchmessers eines bzw. jedes Verbindungsanschlusses. Diese Differenz im Innendurchmesser ist notwendig, da die zur Verteilkammer 4 hinführende Reinigungsflüssigkeitszuführungsleitung 7 eine Art "Energiespeicher" darstellt, sämtliche Verbindungsanschlüsse 9 aber zur Erzielung eines optimierten Reinigungsergebnisses ausreichend mit Reinigungsflüssigkeit versorgt werden müssen. Durch die unterschiedliche Innendurchmesserausgestaltung wird innerhalb der Verteilkammer 4 die Erzeugung eines Staudrucks erreicht.

Die Druckluft wird mit einem Luftdruck von typischerweise 5 bar gefördert. Nach einem Eintrag der Druckluft in die Reinigungsflüssigkeitszuführungsleitung 7 kommt es zeitweise zu einem Anstieg innerhalb der Reinigungsflüssigkeit, der oberhalb des Luftdrucks der eingetragenen Druckluft liegt. Eine solche Druckspitze kann bis zu 150 % des Luftdrucks der eingetragenen Druckluft betragen, was in vorteilhafter Weise zu gegenüber dem Stand der Technik verbesserten Reinigungsergebnissen führt, da auch hartnäckige Verkrustungen oder Verschmutzungen an der Innenoberfläche der zu reinigenden Kanäle, Rohre, Leitungen oder dergleichen sicher entfernt werden können. Dabei wird das Reinigungsergebnis noch dadurch unterstützt, dass durch die Druckluftpulsung ein ZweiPhasen-Reinigungsmedium aus Reinigungsflüssigkeit einerseits und Druckluft andererseits durch die zu reinigenden Kanäle, Rohre, Leitungen oder dergleichen hindurchgeführt wird, was aufgrund seiner Phasengrenzen mechanisch auf zu entfernende Verunreinigungen einwirkt.

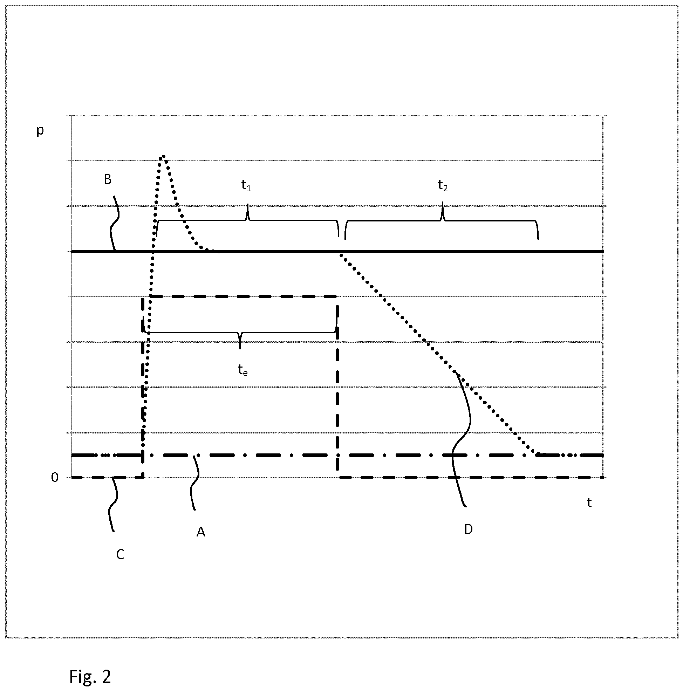

Fig. 2 lässt anhand einer Diagrammdarstellung die erfindungsgemäße Verfahrensdurchführung anhand einer ersten Ausführungsform erkennen. Es ist der Druck p sowie die Ventilansteuerung über der Zeit t dargestellt.

Der mit A gekennzeichnete Graph stellt den Flüssigkeitsdruck der Reinigungsflüssigkeit dar, den sogenannten Spüldruck. Dieser beträgt beispielsweise 0,5 bar.

Der mit B gekennzeichnete Graph meint den Druck der zugeführten Luft. Dieser beträgt beispielsweise zwischen 2 bar und 15 bar, in Krankenhäusern typischerweise 5 bar.

Der mit C bezeichnete Graph stellt die Ansteuerung des Einlassventils 19 für die Druckluft dar. Dieses wird zu einem bestimmten Zeitpunkt schlagartig geöffnet und für eine Zeitspanne t_{E} offengehalten, bis es dann wieder abgeschaltet wird.

Der mit D bezeichnete Graph stellt den Druckverlauf innerhalb der an die Verteilkammer 4 angeschlossenen Kanäle, Rohre, Leitungen oder dergleichen dar. Dieser Druck entspricht zunächst dem anfänglichen Flüssigkeitsdruck von beispielsweise 0,5 bar. Zu dem Zeitpunkt, zu dem das Einlassventil 19 geöffnet und Druckluft in die Reinigungsflüssigkeitszuführungsleitung 7 einströmt, steigt der Druck schlagartig an, und zwar auf einen Druck, der oberhalb des Drucks der Druckluft liegt. Im gezeigten Ausführungsbeispiel beträgt diese Druckspitze beispielsweise 8 bar bis 9 bar.

Nachdem sich die Druckspitze wieder abgebaut hat, wird ein Druck gehalten, der dem der Druckluft entspricht. Dieser Druck wird bis zum Ende der Zeitspanne t_{E}, das heißt der Öffnungsdauer des Einlassventils 19 gehalten. Damit steht innerhalb der zu reinigenden Kanäle, Rohre, Leitungen oder dergleichen für die Zeitdauer t₁ ein Druck an, der größer oder gleich dem Druck der Druckluft ist.

Sobald das Einlassventil 19 geschlossen ist, fällt der Druck innerhalb der Kanäle, Rohre, Leitungen oder dergleichen allmählich ab, bis wieder der Ausgangsdruck, das heißt der Spüldruck der Reinigungsflüssigkeit erreicht ist. Für den Abbau des Druckniveaus wird die Zeitspanne t₂ benötigt.

Solange die Druckdifferenz zwischen dem Druck an den zu reinigenden Kanälen, Rohren, Leitungen oder dergleichen und dem Spüldruck nicht null beträgt, bis also die Zeitspanne t₂ nicht abgelaufen ist, kann kein erneutes Öffnen des Einlassventils 19 erfolgen. Dies deshalb nicht, weil es ansonsten zu ungewollten Rückströmeffekten innerhalb der Reinigungsflüssigkeitszuführungsleitung 7 kommen kann und/oder weil ein zu früh folgender Druckluftimpuls zumindest keine Beaufschlagung der zu reinigenden Kanäle, Rohre, Leitungen oder dergleichen mit Spülflüssigkeit bewirken kann.

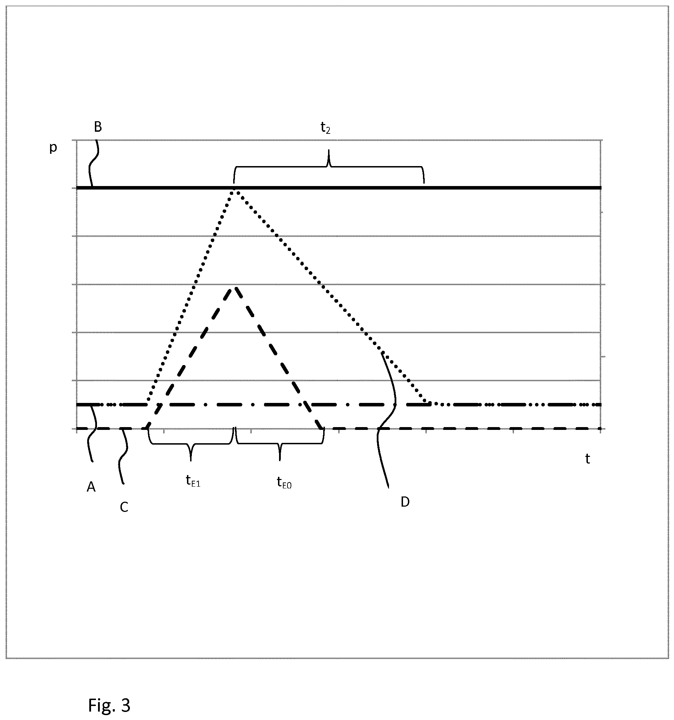

Die Ausführungsvariante nach Fig. 3 unterscheidet sich von der derjenigen nach Fig. 2 dadurch, dass die Druckluft nicht schlagartig sondern durch kontinuierliches Öffnen des Einlassventils 19 allmählich in die Reinigungsflüssigkeitszuführungsleitung 7 eingelassen wird. Auch das Schließen des Einlassventils 19 erfolgt nicht ruckartig sondern vielmehr kontinuierlich, so dass sich insgesamt ein dreieckförmiges Ansteuersignal ergibt, wobei die Zeitspanne t_{E1} die Zeit bis zum vollständigen Öffnen des Einlassventils 19 und die Zeitspanne t_{E0} die Zeitspanne bis zum vollständigen Schließen des Einlassventils 19 meint.

In der Konsequenz dieser Ventilansteuerung ergibt sich ein im Unterschied zur Darstellung nach Fig. 2 anderer Druckverlauf innerhalb der angeschlossenen Kanäle, Rohre, Leitungen oder dergleichen gemäß Graph D. Auch dieser verläuft nach Art eines Dreiecks, wobei es im Unterschied zur Verfahrensdurchführung nach Fig. 2 aufgrund des nur kontinuierlichen Öffnens des Einlassventils 19 an einer anfänglichen Druckspitze mangelt.

Die Verfahrensdurchführung nach Fig. 3 eignet sich insbesondere dann, wenn die mechanische Belastung der zu reinigenden Kanäle, Rohre, Leitungen oder dergleichen gering gehalten werden soll.

### Bezugszeichen

- 1: Reinigungsvorrichtung
- 2: Gehäuse
- 3: Spülraum
- 4: Verteilkammer
- 5: Mediumseingang
- 6: Mediumsausgang
- 7: Reinigungsflüssigkeitszuführungsleitung
- 8: Druckluftzuführungsleitung
- 9: Verbindungsanschluss
- 10: Sammeltopf
- 11: Filter
- 12: Pumpe
- 13: Filter
- 14: Rückschlagventil
- 15: Filter
- 16: Filter
- 17: Pfeil
- 18: Druckluftquelle
- 19: Einlassventile
- 20: Rückschlagventil
- 21: Pfeil
- 22: Einmündung
- A: Graph Flüssigkeitsdruck
- B: Graph Druckluft
- C: Graph Ansteuerung Anlassventil 19
- D: Graph Druck in dem zu reinigenden Kanälen, Rohren, Leitungen oder dergleichen

## Patentansprüche

1. Reinigungsvorrichtung für englumige Kanäle, Rohre, Leitungen oder dergleichen, mit einem Spülraum (3), einer einen Mediumseingang (5) und einen Mediumsausgang (6) aufweisenden Verteilkammer (4), einer Reinigungsflüssigkeitszuführungsleitung (7) und einer Druckluftzuführungsleitung (8), wobei die Verteilkammer (4) eingangsseitig an die Reinigungsflüssigkeitszuführungsleitung (7) angeschlossen ist, und die Verteilkammer (4) ausgangsseitig Verbindungsanschlüsse (9) zur auswechselbaren Anordnung von zu reinigenden englumigen Kanälen, Rohren, Leitungen oder dergleichen aufweist, wobei die Druckluftzuführungsleitung (8) in die Reinigungsflüssigkeitszuführungsleitung (7) einmündet, **dadurch gekennzeichnet dass** die Spülkammer (3) die Verteilkammer (4) aufnimmt und die Verbindungsanschlüsse (9) jeweils einen gegenüber dem Durchströmungsquerschnitt der Reinigungsflüssigkeitszuführungsleitung (7) verringerten Durchströmungsquerschnitt aufweisen so dass innerhalb der Verteilkammer (4) ein Staudruck erzeugt werden kann.

2. Reinigungsvorrichtung nach Anspruch 1, **dadurch gekennzeichnet, dass** die Druckluftzuführungsleitung (8) ein Rückschlagventil (20) aufweist, das in Durchströmungsrichtung der Druckluftzuführungsleitung (8) vor der Einmündung (22) der Druckluftzuführungsleitung (8) in die Reinigungsflüssigkeitszuführungsleitung (7) positioniert ist.

3. Reinigungsvorrichtung nach Anspruch 1 oder 2, **dadurch gekennzeichnet, dass** die Reinigungsflüssigkeitszuführungsleitung (7) ein Rückschlagventil (14) aufweist, das in Durchströmungsrichtung der Reinigungsflüssigkeitszuführungsleitung (7) vor der Einmündung (22) der Druckluftzuführungsleitung (8) in die Reinigungsflüssigkeitszuführungsleitung (7) positioniert ist.

4. Reinigungsvorrichtung nach einem der vorhergehenden Ansprüche, **dadurch gekennzeichnet, dass** die Druckluftzuführungsleitung (8) ein Einlassventil (19) aufweist, das in Durchströmungsrichtung der Druckluftzuführungsleitung (8) dem Rückschlagventil (20) der Druckluftzuführungsleitung (8) vorgeschaltet ist.

5. Reinigungsvorrichtung nach einem der vorhergehenden Ansprüche, **dadurch gekennzeichnet, dass** die Reinigungsflüssigkeitszuführungsleitung (7) einen Drucksensor aufweist, der in Durchströmungsrichtung der Reinigungsflüssigkeitszuführungsleitung nach der Einmündung (22) der Druckluftzuführungsleitung (8) in die Reinigungsflüssigkeitszuführungsleitung (7) positioniert ist.

6. Verfahren zur Reinigung von englumigen Kanälen, Rohren, Leitungen oder dergleichen, bei dem die zu reinigenden englumigen Kanäle, Rohre, Leitungen oder dergleichen mittels einer gemeinsamen Verteilkammer (4) gleichzeitig mit einer einen vorgebbaren Flüssigkeitsdruck aufweisenden Reinigungsflüssigkeit beschickt werden, und dabei in die die Reinigungsflüssigkeit führende Reinigungsflüssigkeitszuführungsleitung über die Dauer einer vorgebbaren Zeitspanne Druckluft mit einem oberhalb des Flüssigkeitsdruck liegenden Luftdruck eingetragen wird wobei ein wiederholter Drucklufteintrag in die die Reinigungsflüssigkeit führende Reinigungsflüssigkeitszuführungsleitung (7) erfolgt, sobald der infolge des vorangegangenen Drucklufteintrags erhöhte Flüssigkeitsdruck wieder abgebaut ist und die zur Verteilkammer (4) hinführende Reinigungsflüssigkeitszuführungsleitung (7) einen größeren Innendurchmesser hat als die von der Verteilkammer (4) abführenden Verbindungsanschlüsse (9) zur auswechselbaren Anordnung der zu reinigenden englumigen Kanälen, Rohren, Leitungen oder dergleichen so dass innerhalb der Verteilkammer (4) ein Staudruck erzeugt werden kann.

7. Verfahren nach Anspruch 6, **dadurch gekennzeichnet, dass** der Flüssigkeitsdruck gemessen und eine Druckdifferenz ermittelt wird.

## Claims

1. Cleaning device for narrow-necked channels, tubes, lines or the like, comprising a rinsing chamber (3),
a distribution chamber (4) that has a medium inlet (5) and a medium outlet (6), a cleaning fluid supply line (7) and a compressed air supply line (8), the distribution chamber (4) being connected on the inlet side to the cleaning fluid supply line (7), and the distribution chamber (4) comprising, on the output side, connection terminals (9) for replaceably arranging narrow-necked channels, tubes, lines or the like to be cleaned, the compressed air supply line (8) opening into the cleaning fluid supply line (7), **characterised in that** the rinsing chamber (3) accommodates the distribution chamber (4) and the connection terminals (9) each have a flow cross section that is reduced relative to the flow cross section of the cleaning fluid supply line (7), such that a dynamic pressure can be produced inside the distribution chamber (4).

2. Cleaning device according to claim 1, **characterised in that** the compressed air supply line (8) comprises a check valve (20) that is positioned, in the direction of flow of the compressed air supply line (8), upstream of the opening (22) of the compressed air supply line (8) into the cleaning fluid supply line (7).

3. Cleaning device according to either claim 1 or claim 2, **characterised in that** the cleaning fluid supply line (7) comprises a check valve (14) that is positioned, in the direction of flow of the cleaning fluid supply line (7), upstream of the opening (22) of the compressed air supply line (8) into the cleaning fluid supply line (7).

4. Cleaning device according to any of the preceding claims, **characterised in that** the compressed air supply line (8) comprises an inlet valve (19) that is arranged upstream of the check valve (20) of the compressed air supply line (8) in the direction of flow of the compressed air supply line (8).

5. Cleaning device according to any of the preceding claims, **characterised in that** the cleaning fluid supply line (7) comprises a pressure sensor that is positioned, in the direction of flow of the cleaning fluid supply line, downstream of the opening (22) of the compressed air supply line (8) into the cleaning fluid supply line (7).

6. Method for cleaning narrow-necked channels, tubes, lines or the like, in which the narrow-necked channels, tubes, lines or the like to be cleaned are simultaneously filled, by means of a common distribution chamber (4), with a cleaning fluid having a predeterminable fluid pressure, and in the process, compressed air having an air pressure that is higher than the fluid pressure is fed, over a predeterminable period of time, into the cleaning fluid supply line conveying the cleaning fluid, wherein compressed air is fed into the cleaning fluid supply line (7) conveying the cleaning fluid again as soon as the fluid pressure that had increased due to the preceding feeding of the compressed air is relieved and the cleaning fluid supply line (7) leading to the distribution chamber (4) has a larger inner diameter than the connection terminals (9) leading away from the distribution chamber (4) for replaceably arranging the narrow-necked channels, tubes, lines or the like to be cleaned, such that a dynamic pressure can be produced inside the distribution chamber (4).

7. Method according to claim 6, **characterised in that** the fluid pressure is measured and a pressure difference is determined.

## Revendications

1. Dispositif de nettoyage pour canaux, tubes, conduites ou similaires à goulots étroits, comprenant un espace de lavage (3),
une chambre de répartition (4) comprenant une entrée de milieu (5) et une sortie de milieu (6), une conduite d'amenée de liquide de nettoyage (7) et une conduite d'amenée d'air comprimé (8), la chambre de répartition (4) étant raccordée côté entrée à la conduite d'amenée de liquide de nettoyage (7), et la chambre de répartition (4) comprenant côté sortie des raccords de liaison (9) servant à l'agencement interchangeable de canaux, tubes, conduites ou similaires à goulots étroits à nettoyer, la conduite d'amenée d'air comprimé (8) débouchant dans la conduite d'amenée de liquide de nettoyage (7), **caractérisé en ce que** la chambre de lavage (3) reçoit la chambre de répartition (4) et les raccords de liaison (9) présentent respectivement une section transversale d'écoulement réduite par rapport à la section transversale d'écoulement de la conduite d'amenée de liquide de nettoyage (7), de telle sorte qu'une pression dynamique puisse être produite à l'intérieur de la chambre de répartition (4).

2. Dispositif de nettoyage selon la revendication 1, **caractérisé en ce que** la conduite d'amenée d'air comprimé (8) comprend une soupape anti-retour (20) qui est positionnée, dans le sens d'écoulement de la conduite d'amenée d'air comprimé (8), avant le débouché (22) de la conduite d'amenée d'air comprimé (8) dans la conduite d'amenée de liquide de nettoyage (7).

3. Dispositif de nettoyage selon la revendication 1 ou 2, **caractérisé en ce que** la conduite d'amenée de liquide de nettoyage (7) comprend une soupape anti-retour (14) qui est positionnée, dans le sens d'écoulement de la conduite d'amenée de liquide de nettoyage (7), avant le débouché (22) de la conduite d'amenée d'air comprimé (8) dans la conduite d'amenée de liquide de nettoyage (7).

4. Dispositif de nettoyage selon l'une des revendications précédentes, **caractérisé en ce que** la conduite d'amenée d'air comprimé (8) comprend une soupape d'admission (19) qui est placée en amont de la soupape anti-retour (20) de la conduite d'amenée d'air comprimé (8) dans le sens d'écoulement de la conduite d'amenée d'air comprimé (8).

5. Dispositif de nettoyage selon l'une des revendications précédentes, **caractérisé en ce que** la conduite d'amenée de liquide de nettoyage (7) comprend un capteur de pression qui est positionné, dans le sens d'écoulement de la conduite d'amenée de liquide de nettoyage, après le débouché (22) de la conduite d'amenée d'air comprimé (8) dans la conduite d'amenée de liquide de nettoyage (7).

6. Procédé de nettoyage de canaux, tubes, conduites ou similaires à goulots étroits, selon lequel les canaux, tubes, conduites ou similaires à goulots étroits à nettoyer sont alimentés simultanément, au moyen d'une chambre de répartition (4) commune, en un liquide de nettoyage présentant une pression de liquide pouvant être prédéfinie et en l'occurrence de l'air comprimé présentant une pression d'air supérieure à la pression de liquide est introduit dans la conduite d'amenée de liquide de nettoyage guidant le liquide de nettoyage pendant la durée d'une période pouvant être prédéfinie, une introduction d'air comprimé répétée dans la conduite d'amenée de liquide de nettoyage (7) guidant le liquide de nettoyage s'effectuant dès que la pression de liquide accrue suite à l'introduction d'air comprimé précédente est diminuée à nouveau, et la conduite d'amenée de liquide de nettoyage (7) menant jusqu'à la chambre de répartition (4) ayant un plus grand diamètre intérieur que les raccords de liaison (9), partant de la chambre de répartition (4), servant à l'agencement interchangeable des canaux, tubes, conduites ou similaires à goulots étroits à nettoyer, de telle sorte qu'une pression dynamique soit produite à l'intérieur de la chambre de répartition (4).

7. Procédé selon la revendication 6, **caractérisé en ce que** la pression de liquide est mesurée et une différence de pression est déterminée.
